# EUROPÄISCHE PATENTSCHRIFT

(11) **EP 1 141 373 B1**
(45) Veröffentlichungstag und Bekanntmachung des Hinweises auf die Patenterteilung: **17.05.2006**
(21) Anmeldenummer: 99964440.4
(22) Anmeldetag: 08.12.1999
(51) Int. Cl.: C12P 41/00, C12P 7/42, C12P 7/40, C12P 7/62, C12R 1/225, C12R 1/24

(54) **VERFAHREN ZUR ENANTIOSELEKTIVEN UND REGIOSELEKTIVEN REDUKTION VON 3,5-DIOXOCARBONSÄUREN, DEREN SALZE UND ESTER**
METHOD FOR THE ENANTIOSELECTIVE AND REGIOSELECTIVE REDUCTION OF 3,5-DIOXOCARBOXYLIC ACIDS, THEIR SALTS AND THEIR ESTERS
PROCEDE DE REDUCTION ENANTIOSELECTIVE ET REGIOSELECTIVE D'ACIDES 3,5-DIOXOCARBOXYLIQUES, LEURS SELS ET ESTERS

(30) Priorität: 14.12.1998 DE 19857302
(43) Veröffentlichungstag der Anmeldung: 10.10.2001
(73) Patentinhaber: Forschungszentrum Jülich GmbH, 52425 Jülich (DE)
(72) Erfinder: WOLBERG, Michael, D-52428 Jülich (DE); MÜLLER, Michael, D-52428 Jülich (DE); HUMMEL, Werner, D-52445 Titz (DE)
(86) Internationale Anmeldenummer: PCT/DE1999/003971
(87) Internationale Veröffentlichungsnummer: WO 2000/036134

(56) Entgegenhaltungen:
- EP-A- 0 569 998
- WO-A-97/00968
- DE-A- 19 610 984
- PATEL R. N. ET AL.: "Enantioselective microbial reduction of 3,5-dioxo-6-(benzyloxy) hexanoic acid, ethyl ester." ENZYME MICROB. TECHNOL., Bd. 15, Nr. 12, 1993, Seiten 1014-1021, XP000651790 in der Anmeldung erwähnt
- PETERS J. ET AL.: "Studies on the distribution and regulation of microbial keto ester reductases." APPLIED MICROBIOLOGY AND BIOTECHNOLOGY, Bd. 38, 1992, Seiten 334-340, XP002134909

## Beschreibung

Die Erfindung betrifft ein Verfahren zur r-selektiven und regioselektiven Reduktion in Position 5 von 3,5-Dioxocarbonsäuren sowie deren Salze und Ester.

Homochirale 3,5 -Dihydroxycarbonsäurederivate der Formel 1 sind Intermediate in der Synthese einer Vielzahl von Natur- und Wirkstoffen.

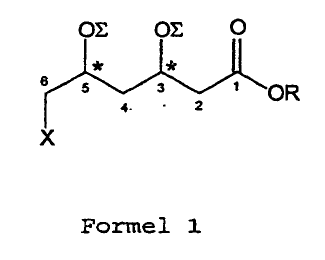

X = eine Komponente aus der Gruppe Wasserstoff, Halogen, Alkyl, Aryl, CH=CHR², C≡CR³ (mit R²=R außer Metallkation und R³=R)

Σ: H oder Schutzgruppe für die Hydroxylfunktion.

R: H, Metallkation, Alkyl-, Aryl-, Aralkyl oder Cycloalkyl-Rest.

Je nach absoluter Konfiguration an den Stereozentren C-3 und C-5 können sie zielgerichtet bei der Synthese chiraler Naturstoffe, wie Mevinsäuren oder synthetischer HMG-CoA-Reduktase-Inhibitoren eingesetzt werden.

Andere Natur- oder Wirkstoffe verlangen andere Konfigurationen der stereogenen Zentren in Position C-3 und C-5. Es besteht daher ein großes Interesse, alle möglichen Stereoisomere der 3,5-Dihydroxycarbonsäurederivate nach Formel 1 in optisch reiner Form darstellen zu können. Eine vorteilhafte Methode zur Herstellung dieser Verbindungen ist die katalytische enantioselektive Reduktion der prochiralen 3,5-Dioxocarbonsäurederivaten nach Formel 2.

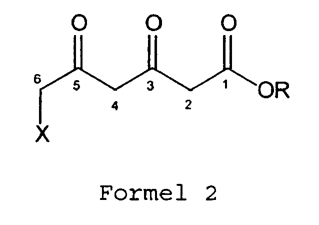

Bei Anwendung dieser Methode entfallen kostenintensive und umweltbelastende Racemat-, Scalemat- oder Diastereomerentrennungen. Die in einer diastereoselektiven Synthese erforderliche Anbindung und Abspaltung einer stöchimetrischen Menge einer homochiralen Hilfsgruppe wird vermieden. Weiterhin ist das Kohlenstoffgerüst der 3,5-Dihydroxycarbonsäureester nach Formel 1 in den Ausgangsverbindungen nach Formel 2 bereits komplett, d. h. die Stereozentren werden erst zu einem späten Zeitpunkt in der Gesamtsynthesesequenz eingeführt, wodurch der Verlust an homochiralem Material niedrig gehalten wird.

Die europäische Patentanmeldung 0 569 998 A2 offenbart ein mikrobielles Verfahren zur Reduktion von in 6-Position oxyalkyl- und oxyaralkylsubstituierten Diketoestern, welches enantioselektiv ist.

Die WO 97/00968 offenbart ein Verfahren zur Reduktion von 3-Oxo-5-hydroxy-Carbonsäureestern mittels Reduktasen von Beauveria, Candida, Kluyveromycis, Torulaspora oder Pichia.

Die DE 196 10 984 A1 offenbart ein stabiles mikrobielles Enzym mit Alkohol-Dehydrogenase-Aktivität; ein Verfahren zu dessen Gewinnung sowie dessen Verwendung zur enantioselektiven Reduktion/Oxidation von organischen Ketoverbindungen/Hydroxyverbindungen, wobei je nach Art der Ausgangsverbindungen R- oder S-Hydroxyverbindungen erhalten werden.

Die Veröffentlichung "Enantioselective microbial reduction of 3,5-dioxo-6-(benzyloxyl)hexanoic acid, ethyl ester" in Enzyme Microb. Technol. (1993), 15(12), 1014-21 ff zeigt die Reduktion von 3,5-Dioxo-6-(benzyloxy)hexansäure-Ethylester mittels einer Reduktase.

Die Veröffentlichung "Studies on the distribution and regulation of microbial keto ester reductases" in Appl. Micobiol. Biotechnol. (1992), 38, 334- 340, zeigt die Reduktion von Ketosäuren und Estern mittels einer Reduktase aus Acetobacter, Alcaligenes, Gluconobacter, Methylomonas, Pseudomonas, Rhodococcus und Hefen. Aus terminologischen Gründen soll hier eine Begriffsdefinition als Arbeitsbegriff eingeführt werden, welche innerhalb der Offenbarung Gültigkeit haben soll:

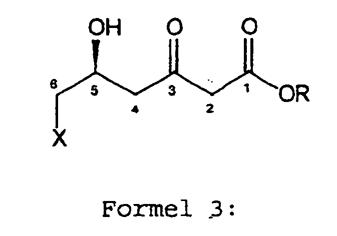

In Formel 3 tritt die OH-Gruppe in 5-Position aus der Papierebene hervor, während die Ketten mit der Carbonsäurefunktion bzw. Esterfunktion sowie mit dem Rest X in der Papierebene liegen. Das Wasserstoffatom an C-5 fällt somit hinter die Papierebene. Nach der CIP (RS)-Nomenklatur würde für dieselbe räumliche Konfiguration der OH-Gruppe aus der Papierebene nach vorne je nach Priorität der Kette welche mit X substituiert ist eine R- oder S- Bezeichnung verwendet werden. Im Sinne der Erfindung soll nun für eine Anordnung der Substituenten, bei der die OH-Gruppe in 5-Position aus der Papierebene nach vorne heraustritt, der Wasserstoff in 5-Position unter die Papierebene tritt und bei der die mit der Carbonsäure- oder Esterfunktion ausgestattete Seitenkette auf der rechten und die mit dem Substituenten X augestattete Seitenkette auf der linken Seite des fünften C-Atoms liegt, die Bezeichnung r-Konfiguration benutzt werden. Für die Fälle in denen die mit der Carbonsäurefunktion oder Esterfunktion ausgestattete rechte Seitenkette eine höhere Priorität hat als die mit dem Substituenten X ausgestattete linke Seitenkette entspricht dies der klassischen R-Konfiguration. Sind die Prioritäten der eben genannten Ketten (z.B. durch die Wahl X = Halogen) vertauscht so würde der Zielverbindung die klassische S-Konfiguration zugeschrieben werden. Beide Fälle sollen jedoch von der Definition r-Konfiguration umfaßt werden.

Es ist die Aufgabe der Erfindung bei einer enzymatischen Reduktion von 3,5-Dioxocarbonsäurederivaten regioselektiv in der 5-Position eine r-Konfiguration der Hydroxylgruppe einzuführen.

Weiterhin ist es die Aufgabe der Erfindung ein neues Verfahren zur Bereitstellung enantiomerenreiner 3,5-Dihydroxycarbonsäurederivate zur Verfügung zu stellen, also ein Verfahren zu schaffen, mit dem in der 3-Position zur 5-Position zielgerichtet eine syn- oder anti-Reduktion der Ketogruppe erfolgen kann, so daß das 3,5-Dihydroxycarbonsäurederivat eine maßgeschneiderte absolute Konfiguration in Bezug auf C-3 aufweist, die wunschgemäß hergestellt werden kann.

Weiterhin ist es die Aufgabe der Erfindung ein verbessertes Verfahren zur Synthese von 3,5-Dioxocarbonsäureestern nach Formel 4 zur Verfügung zu stellen, die als Edukte für die enzymatische Reduktion eingesetzt werden können.

Ausgehend vom Oberbegriff des Anspruchs 1 wird die Aufgabe erfindungsgemäß gelöst mit den im kennzeichnenden Teil des Anspruchs 1 angegebenen Merkmalen.

Mit dem erfindungsgemäßen Verfahren ist es nunmehr möglich, 3,5-Dioxocarbonsäuren sowie deren Ester hochenan-tioselektiv und regioselektiv in Position 5 zu reduzieren und somit Verbindungen zu erhalten, welche weiterhin in einer Synthese von Natur- und Wirkstoffen eingesetzt werden können, die in dem 3,5-Dihydroxycarbonsäure-Strukturelement eine definierte absolute Konfiguration haben.

Vorteilhafte Weiterbildungen der Erfindung sind in den Unteranspürchen angegeben.

Im folgenden soll die Erfindung in allgemeiner Form beschrieben werden.

Erfindungsgemäß wird eine Verbindung gemäß Formel 4 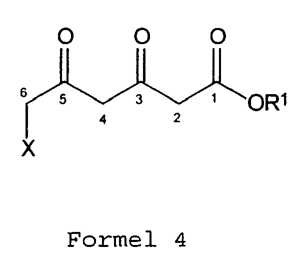 mit R¹ = eine Komponente aus der Gruppe Alkyl, Alkenyl, Cycloalkyl, Cycloalkenyl, Aryl, Aralkyl, Cycloalkylalkyl, Wasserstoff oder Metallkation und
X = eine Komponente aus der Gruppe Wasserstoff, Halogen, Alkyl, Aryl, CH=CHR², C≡CR³ (mit R²=R¹ außer Metallkation und R³=R¹) mittels einer Alkoholdehydrogenase unter Zusatz von NADPH oder eines anderen Cofaktors umgesetzt.

Unter Alkyl sind sowohl geradkettige als auch verzweigte gesättigte Kohlenstoffketten zu verstehen. Beispielhaft können Methyl, Ethyl, n-Propyl, i-Propyl, t-Butyl, Pentyl, i-Pentyl, n-Hexyl, i-Hexyl genannt werden. Mit Alkenyl sind geradkettige und verzweigte ungesättigte Kohlenwasserstoffe bezeichnet, für die beispielhaft Vinyl, 1-Propenyl, Allyl, Butenyl, i-Butenyl genannt werden können. Von dem Begriff Cycloalkyl werden gesättigte ringförmige Kohlenwasserstoffketten umfaßt, die aus drei, vier, fünf, sechs oder sieben Kohlenstoffatomen bestehen. Cycloalkenyl bezeichnet ungesättigte ringförmige Kohlenwasserstoffe, mit 5,6,7 oder 8 Kohlenstoffatomen. Unter Aryl sind aromatische Systeme, eingeschlossen Heteroaromaten und substituierte aromatische Systeme, wie Phenyl, p-Tolyl, Furanyl zu verstehen. Unter Aralkyl sind Arylreste zu verstehen, die über Alkylgruppen angebunden sind, wie zum Beispiel ein Benzylrest. Unter den Begriff Cycloalkylalkyl fallen Cycloalkylreste, die über Alkylgruppen gebunden sind. Unter den Halogenen sind Fluor und Chlor bevorzugt.

Die Alkoholdehydrogenase ist bevorzugt rekombinant und stammt aus *Lactobacillus,* insbesondere *Lactobacillus brevis* (rekLBADH). Besonders vorteilhaft an diesem Enzym ist, daß es rekombinant überexprimiert werden und somit in großen Mengen verfügbar gemacht werden kann. Dies eröffnet auch den Einsatz in größerem technischem Maßstab. Die Reaktion kann dabei sowohl in wäßrigem Medium mittels des Enzyms, als auch intrazellulär in einem Mikroorganismus stattfinden. In einer bevorzugten Ausführungsform wird die rekLBADH aus *Lactobacillus brevis* eingesetzt, die rekombinant in *Escherichia coli* überexprimiert wurde.

Ein weiterer Vorteil der rekLBADH ist die Eigenschaft des Enzyms, den benötigten Cofaktor NADPH substratgekoppelt regenerieren zu können, das heißt, NADPH muß nicht in stöchiometrischer Menge eingesetzt werden. Weiterhin kann damit die Zugabe eines zweiten Enzyms zur Regeneration des NADPH vermieden werden. Hierdurch werden letztendlich auch Kosten gesenkt. Bei dieser Transferhydrierung kann als Wasserstoffdonor ein Alkohol, bevorzugt Isopropanol eingesetzt werden. Die Aktivität der rekLBADH kann durch die Zugabe von Mg²⁺ gesteigert werden.

Das erfindungsgemäße Verfahren kann bei Raumtemperatur durchgeführt werden, da das Enzym rekLBADH vorteilhafterweise eine hohe Thermostabilität aufweist. Dies hat auch zur Folge, daß für einen gewünschten Umsatz weniger Enzym benötigt wird, wodurch Kosten gespart werden können. Auf aufwendige Kühlmaßnahmen kann verzichtet werden. Das erfindungsgemäße Verfahren kann jedoch auch bei für enzymatische Reaktionen üblichen Temperaturen zwischen 0°C und 70°C durchgeführt werden. Bevorzugt ist der Bereich zwischen 20°C und 50°C.

Das erfindungsgemäße Verfahren kann bei einem pH-Wert von 5,5 durchgeführt werden, da das bevorzugte Enzym bei pH 5,5 ein Stabilitätsmaximum aufweist. Dies ist von Vorteil, da viele Estersubstrate nach Formel 4 bei diesem pH-Wert eine höhere Stabilität aufweisen als bei höheren pH-Werten, die normalerweise in enzymatischen Reaktionen eingehalten werden müssen. Die Reaktion kann jedoch auch in einem pH-Wert-Bereich von 5,5 bis 9 durchgeführt werden, bevorzugt in einem Bereich zwischen 5,5 bis 6,5.

Zur Sicherstellung eines geeigneten pH-Wertes kann jede für eine enzymatische Reaktion geeignete Puffersubstanz eingesetzt werden. Diese sind zum Beispiel: Triethanolamin (TEA), Phosphatpuffer oder TRIS-Puffer. Die Konzentrationsbereiche für die Puffer liegen vorteilhafterweise zwischen 50 und 500 mmol/l.

Das Reaktionsprodukt mit der r-Konfiguration ist in Formel 5 wiedergegeben, in der die Substituenten R¹ und X dieselbe Bedeutung haben, wie in Formel 4.

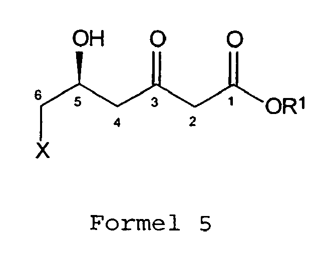

Die Enantiomerenüberschüsse können für diese Reaktionen mit ≥ 98%-100% angegeben werden.

In einer Fortbildung der Erfindung wird die Ketogruppe der Verbindung in Formel 5 in Position 3 diastereoselektiv zu einer OH-Gruppe reduziert, welche dann in syn- oder anti-Stellung zur OH-Gruppe in 5-Position steht.

Die Umsetzung der Verbindung nach Formel 5 zu dem Reaktionsprodukt 6a,b kann mit Methoden erfolgen, die für die Synthese von *syn-* (6a) und *anti-*Diolen (6b) bekannt sind.

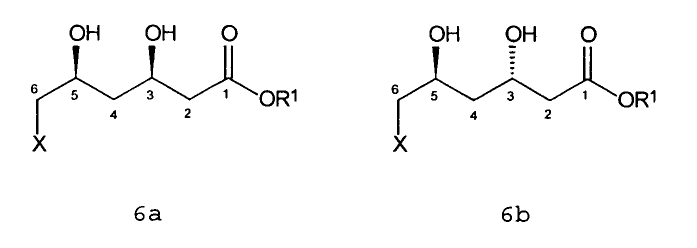

Diese sind für die Herstellung von *syn*-Diolen (6a) aus β-Hydroxycarbonylverbindungen zum Beispiel Natriumborhydrid-Reduktion in Gegenwart von Trialkyl- oder Alkoxydialkylboranen (1: K. Narasaka, F. C. Pai, *Tetrahedron* **1984**, 40, 2233-2238.
2: K. M. Chen, G. E. Hardtmann, K. Prasad, O. Repic, M. J. Shapiro, *Tetrahedron* Lett. **1987**, 28, 155-158.)
und für anti-Diole (6b) aus β-Hydroxycarbonylverbindungen zum Beispiel Reduktion mit Tetramethylammoniumtriacetoxyborhydrid (D. A. Evans, K. T. Chapman, E. M. Carreira, *J. Am. Chem. Soc.* **1988,** 110, 3560-3578.).

Generell kommen für den zweiten Schritt sowohl chemische als auch enzymatische Reduktionen in Frage. Die enzymatische Reduktion der 3-Position kann beispielsweise mit den folgenden Mikroorganismen oder deren isolierten Reduktasen durchgeführt werden: Beauveria bassiana ATCC 7159, Candida humicola CBS 1897, Candida diddensiae ATCC 20213, Candida frieddrichii ATCC 22970, Candida solani CBS 1908,Hansenula nonfementans CBS 5764, Kluyveromyces drosophilarum CBS 2105, Pichia angusta NCYC 495, Pichia angusta NCYC R320, Pichia angusta NCYC R322, Pichia haplophila CBS 2028, Pichia membranefaciens DSM 70366, Pichia pastoris BPCC 260, Pichia pastoris BPCC 443, Pichia pastoris NCYC R321, Torulaspora hansenii ATCC 20220, Candida pelliculosa ATCC 2149, Hansenula anomola CBS 2230, Neurospora crassa ATCC 9277, Pichia trehalophila CBS 5361, Mortierella alpina MF 5534 (ATCC8979).

Es können auch die Reduktasen aus Saccharomyces, insbesondere Saccharomyces cerevisiae sowohl in der Zelle als auch in isolierter Form eingesetzt werden.

Das erfindungsgemäße Herstellungsverfahren kann vorteilhafterweise in einem kontinuierlichen Prozeß in einem Enzym-Membranreaktor, wie er beispielhaft in der DE-PS 39 37 892 beschrieben ist durchgeführt werden. Die enantioselektive Reduktion von 3,5-Dioxocarbonsäureestern an der C-5-Position kann mit Wildtypenzymen und /oder rekombinant überexprimierten Enzymen und mit ganzen Zellen stattfinden. Bevorzugt ist jedoch die extrazelluläre Umsetzung mit einem Zellrohextrakt, da hierbei höhere Enantiomerenüberschüsse erzielt werden.

In einer vorteilhaften Weiterbildung der Erfindung wird das zur Synthese benötigte Substrat nach einer Methode dargestellt, die im Gegensatz zu anderen etablierten Verfahren zur Synthese von 3,5-Dioxocarbonsäurederivaten nach Formel 4 mit besonders preisgünstigen und einfachen Ausgangsstoffen auskommt. Ein weiterer Vorteil dieses Verfahrens ist die unkomplizierte Reaktionsführung.

Im folgenden wird das erfindungsgemäße Verfahren beschrieben, mit dem 3,5-Dioxocarbonsäureester nach Formel 2 durch Acylierung von Bisenolaten nach Formel A mit R⁴= Alkyl, Alkenyl, Cycloalkyl, Cycloalkenyl, Aryl, Aralkyl, Cycloalkylalkyl oder Metallkation 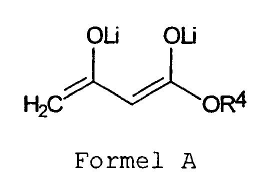 mit leicht zugänglichen und billigen Carbonsäureestern nach Formel B, in welcher X die gleiche Bedeutung wie in Formel 2 hat, und in welcher R⁵ für einen Alkylrest steht, 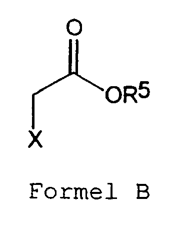 in hohen Ausbeuten dargestellt werden können.

Gemäß bekannter Arbeitsmethoden wird aus einem β-Ketoester mit einer Lithiumverbindung in einem organischen Lösungsmittel *in situ* ein Bisenolat nach Formel A erzeugt. Dieses Bisenolat nach Formel A wird anschließend unter Kontrolle der Innentemperatur des Reaktionsgefäßes durch Zugabe eines Carbonsäureesters nach Formel B acyliert, wobei bevorzugt α-Halogencarbonsäureester zum Einsatz kommen. Besonders bevorzugt sind die Methylester der Chloressigsäure und der Fluoressigsäure. Es erfolgt anschließend eine saure wäßrige Aufarbeitung in Anwesenheit eines organischen Lösungsmittels, das nicht mit Wasser mischbar ist, wie z. B. Essigsäureethylester oder Diethylether.

Die erfindungsgemäße Reaktion wird bevorzugt unter einer Inertgasatmosphäre und wasserfreien Bedingungen durchgeführt. Als Inertgas kommen z. B. Stickstoff oder Argon in Frage. Als Lösungsmittel kommen inerte organische Lösungsmittel wie Ether, Alkane oder Cycloalkane (C₅-C₇), Toluol oder Benzol in Frage. Bevorzugt sind aprotische koordinierende Lösungsmittel aus der Verbindungsklasse der Ether, wie z. B. Diethylether, Dimethoxyethan oder Tetrahydrofuran (THF). THF ist besonders bevorzugt.

Die Lithiumverbindung ist bevorzugt stark basisch, aber wenig nucleophil. Bevorzugt sind Lithiumamide, wie z. B. Lithiumdiisopropylamid (LDA), Lithiumdicyclohexylamid, Lithiumcyclohexylisopropylamid, Lithium-2,2,6,6-tetramethylpiperidin (LiTMP) oder Lithium-bis-(trimethylsilyl)-amid (LiHMDS). LDA ist besonders bevorzugt. Möglich sind auch Organolithiumverbindungen wie z. B. Mesityllithium oder t-Butyllithium. Das bevorzugte molare Verhältnis zwischen Lithiumbase und β-Ketoester zur Erzeugung des Bisenolates ist 2 : 1 bis 4 : 1, besonders bevorzugt ist ein molares Verhältnis von 2,1 : 1.

Die Acylierung des *in situ* erzeugten Bisenolats nach Formel A durch Zugabe eines Carbonsäureesters nach Formel B erfolgt bevorzugt bei einer Innentemperatur des Reaktionsgefäßes von -100° bis +25°C, am günstigsten zwischen -80° und -40°C. Besonders bevorzugt ist der Bereich zwischen -72° und -65°C. Das molare Verhältnis von Carbonsäureester zu Bisenolat liegt bevorzugt zwischen 0,5 : 1 und 2 : 1. Besonders bevorzugt ist ein Verhältnis von 1 : 1.

Die Aufarbeitung des stark basischen Reaktionsgemisches erfolgt bevorzugt zwei bis 120 Minuten nach Zugabe der letzten Portion des Carbonsäureesters nach Formel B, am günstigsten nach 15 - 30 Minuten. Dazu wird der Inhalt des Reaktionsgefäßes auf eine eisgekühlte und kräftig gerührte Mischung aus einem mit Wasser nicht mischbaren organischen Lösungsmittel, wie z. B. Essigsäureethylester oder Diethylether, und einer wäßrigen Lösung einer Säure, wie z. B. verdünnte Chlorwasserstoffsäure, Essigsäure oder Ammoniumchlorid-Lösung, gekippt. Bevorzugt werden hierfür zweimolare Chlorwasserstoffsäure und Essigsäureethylester verwendet.

Diese Vorgehensweise ist im Zusammenhang mit der Acylierung von Bisenolaten nach Formel A mit Carbonsäureestern nach Formel B eine Verbesserung bestehender Methoden, da ein Überschuß an Base bzw. Bisenolat vermieden wird, ebenso wie die Verwendung spezieller teurer Acylierungsreagenzien, Katalysatoren oder Cosolventien. (a) M. Yamaguchi, K. Shibato, H. Nakashima, T. Minami, *Tetrahedron* **1988,** 44, 4767-4775. b) N. S. Narasimhan, R. K. Ammanamanchi, J. *Org. Chem.* **1983,** 48, 3945-3947. c) S. N. Huckin, L. Weiler, *Can.* J. *Chem.* **1974,** 52, 1343-1351.) Ein weiterer Vorteil ist die unkomplizierte Reaktionsführung, da die Komponenten nicht wie bei Huckin et al. in Intervallen abwechselnd zugegeben werden müssen, sondern die benötigten Volumina in einer Portion zugefügt werden können. Mit dem erfindungsgemäßen Verfahren kommt es nicht zur Bildung von nennenswerten Mengen an Nebenprodukten. Mit dem erfindungsgemäßen Verfahren wurden verschiedene 3,5-Dioxocarbonsäureester nach Formel 2 dargestellt, von denen exemplarisch die Verbindungen 6-Chlor-3,5-dioxohexansäure-1,1-dimethylethylester und 6-Fluor-3,5-dioxohexansäure-1,1-dimethylethylester genannt seien. Beide Verbindungen sind neu.

### Beispiele:

Im folgenden wird das erfindungsgemäße Verfahren in bevorzugten Ausführungsformen beispielhaft dargestellt. Jedoch ist das erfindungsgemäße Verfahren nicht auf die Beispiele beschränkt.

### Beispiel 1

### (3R,5S)-6-Chlor-3,5-dihydroxyhexansäure-1,1-dimethyl-ethylester

### a) 6-Chlor-3,5-dioxohexansäure-1,1-dimethylethylester

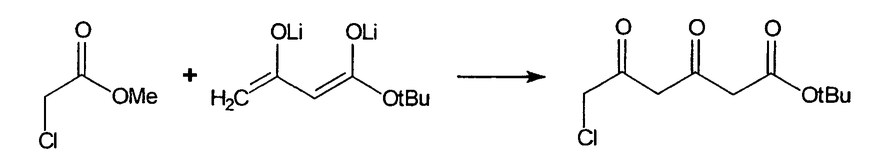

Die folgende Reaktion wird in einer ausgeheizten Standardglasapparatur (Dreihalskolben, Tropftrichter) unter Ausschluß von Feuchtigkeit und Luft durchgeführt (N₂-Schutzgasatmosphäre). Die Apparatur ist mit Septen versehen, durch die die Reagenzien, die allesamt kommerziell erhältlich sind, mittels Kanülen unter Luftausschluß zugegeben werden können. Die im Text erfolgenden Temperaturangaben beziehen sich auf die Innentemperatur des Reaktionsgefäßes. Dementsprechend ist die Apparatur mit einem Innenthermometer versehen. Das Reaktionsgemisch wird zu jedem Zeitpunkt der Reaktionsdurchführung gut gerührt (Magnetrührer).

In die soeben beschriebene Apparatur werden 250 - 300 ml absolut wasserfreies THF und 10,63 g (105 mmol) Diisopropylamin gegeben. Nachdem der Kolbeninhalt mit einem Eis-Kochsalz-Kältebad auf -15°C gekühlt wurde, läßt man über den Tropftrichter 64 ml (ca. 105 mmol) einer n-Butyllithium-Lösung in n-Hexan (ca. 1,6 molar) langsam unter Rühren zutropfen, wobei eine Temperatur von 0°C nicht überschritten werden soll. Nach beendeter Zugabe verbleibt der Kolben im Kältebad und es wird noch 10 Minuten gerührt. Anschließend werden langsam 7,91 g (50 mmol) 3-Oxobutansäure-1,1-dimethylethylester zugetropft, wobei eine Temperatur von -5°C nicht überschritten werden soll. Der Kolben wird nach beendeter Zugabe etwas aus dem Kältebad gehoben, und das Reaktionsgemisch noch 10 Minuten bei -10° bis maximal -5°C gerührt. Hiernach wird das Eis-Kochsalz-Kältebad gegen ein Kältebad ausgetauscht, das aus Aceton, Trockeneis und etwas flüssigen Stickstoff besteht. Nach Abkühlung der Reaktionslösung auf -72°C werden 5,43 g (50 mmol) Chloressigsäuremethylester so langsam zugetropft, daß eine Temperatur von -65°C nicht überschritten wird. Nach beendeter Zugabe läßt man noch 25 Minuten bei Temperaturen zwischen -70°C bis maximal -65°C rühren und kippt die Reaktionslösung anschließend auf eine kräftig gerührte und mit einem Eis-Kältebad auf 4°C gekühlte Mischung aus 150 ml Essigsäureethylester und 150 ml zweimolarer Chlorwasserstoffsäure. Das Gemisch wird unmittelbar danach in einen Scheidetrichter überführt und gut durchgeschüttelt. Die Phasen werden getrennt, und die wäßrige Phase wird noch zweimal mit jeweils 150 ml Essigsäureethylester extrahiert. Die vereinigten organischen Phasen werden mit 150 ml NaHCO₃-Lösung (5 %) gewaschen. Falls es an dieser Stelle Probleme mit der Phasentrennung gibt, werden 40 g Kochsalz zugelöst. Die organische Phase wird anschließend mit 150 ml gesättigter Kochsalz-Lösung gewaschen, mit Natriumsulfat getrocknet, filtriert und im Membranpumpenvakuum am Rotationsverdampfer bei maximal 40°C soweit wie möglich eingeengt. Es verbleiben 11 g öliges Rohprodukt (94%), das gemäß einer ¹H-NMR-Analyse zu ca. 95% aus dem gewünschten Produkt besteht. Ein Dünnschichtchromatogramm (Kieselgel; Essigsäureethylester : i-Hexan 3 : 7; Entwicklung durch Fluoreszenzlöschung oder Bedampfung mit Iod) zeigt geringe Anteile einer polareren und einer unpolareren Verunreinigung.

Die Reinigung des Produktes kann durch Kugelrohrdestillation bei max. 75°C und einem Vakuum von mindestens 0.01 mbar erfolgen. Auf diese Weise wurde aus 1,12 g des Rohproduktes 0,83 g analytisch reines Produkt isoliert. Alternativ kann eine Flash-Chromatographie mit säuregewaschenem, metallfreien Kieselgel durchgeführt werden (kommerziell erhältliches Kieselgel (Standardqualität) wird vor der Chromatographie 24 Stunden in zweimolarer Chlorwasserstoffsäure suspendiert, filtriert, mit deionisiertem Wasser gewaschen bis die Waschflüssigkeit einen pH-Wert von 5.5 aufweist, und anschließend mindestens 24 Stunden bei 105°C und Normaldruck getrocknet. Siehe auch: J. S. Hubbard, T. M. Harris, J. *Org. Chem.* **1981**, 46, 2566-2570.). Mit dieser Methode wurden aus 3,81 g des Rohproduktes 2,72 g analytisch reines Produkt und 0,24 g einer Mischfraktion erhalten (Säule mit 6 cm Durchmesser, 220 g Kieselgel, Essigsäureethylester : i-Hexan 2 : 8, 60 ml Fraktionen). Die Verbindung kann bei -20°C über mehrere Monate gelagert werden.

¹H-NMR (300 MHz, CDCl₃, 22°C) 1.) *Enolform* δ: 14.76 (s, 1H, OH, br), 5.97 (s, 1H, H4), 4.06 (s, 2H, H6), 3.31 (s, 2H, H2), 1.48 (s, 9H, 3 x CH₃) .― 2.) *Ketoform δ:* 4.21 (s, 2H, H6), 3.92 (s, 2H, H4), 3.49 (s, 2H, H2), 1.47 (s, 9H, 3 x CH₃).- *Keto : Enol* = *12* : 88.- ¹³C-NMR (75.5 MHz, CDCl₃, es *werden nur die Signale der Enolform angegeben)* δ: 28.14 (C(CH₃)₃), 44.35 (C6), 46.03 (C2), 82.56 (OC(CH₃)₃), 98.89 (C4), 166.48 (COO*t*Bu), 187.05, 187.34 (C3, C5).

### b) (S)-6-Chlor-5-hydroxy-3-oxohexansäure-1,1-dimethyl-ethylester

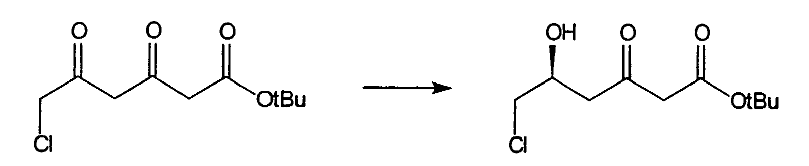

Die folgende enzymatische Reaktion wird mit der Oxidoreduktase rekLBADH durchgeführt. Es wird ein Zellrohextrakt verwendet, der durch Naßvermahlung von Feuchtzellmasse eines rekombinanten E. *coli* Stammes (recADHHB101+) hergestellt wird. Die Kultivierungsbedingungen und die Einzelheiten des Zellaufschlusses sind in der Literatur beschrieben (B. Riebel, *Disserta*tion, Heinrich-Heine-Universität-Düsseldorf, **1996;** siehe auch: *Deutsche Patentanmeldung 19610984.1,* **1996).** Aus 1 g Feuchtzellmasse werden nach dieser Methode ca. 3 ml Zellrohextrakt erhalten. Die Aktivität dieser Enzympräparation beträgt ca. 1100 U/ml unter folgenden Bedingungen: photometrische Bestimmung bei 340 nm (ε_{NADPH} = 6.22 cm²/µmol); Substrat: Acetophenon (10 mM), NADPH (0,25 mM), MgCl₂ (1 mM), Phosphat-Puffer (100 mM, pH 6,5), limitierende Enzymmenge; 1 ml Gesamtvolumen; Temperatur: 25°C; Messung über 1 Minute. Eine Enzymeinheit (U) ist definiert als die Enzymmenge, die 1 µmol NADPH pro Minute unter den angegebenen Bedingungen oxidiert.

Im Folgenden wird die einfachste Form der Reaktionsführung beschrieben (Satzreaktor, einphasig). Als Reaktionsgefäß dient ein Rundkolben geeigneter Größe. Während der enzymatischen Reduktion wird der Kolbeninhalt langsam (ca. 60 rpm) mit einem Magnetrührer gerührt.

Zu 470 ml Phosphat-Citrat-Puffer (250 mM Na₂HPO₄, 125 mM Citronensäure, mit NaOH auf pH 5,5 eingestellt) wird eine Lösung aus 2,21 g (9,4 mmol) des in Schritt a) hergestellten 6-Chlor-3,5-dioxohexansäure-1,1-dimethyl-ethylester in 5,66 g (94 mmol) Isopropanol (zur Cofaktorregenerierung) gegeben. Zur Beschleunigung des Lösungsvorganges kann eine kurze Ultraschallbehandlung angewendet werden. Weiterhin werden zu dieser Lösung 1,92 g (9,4 mmol) Magnesiumchlorid-Hexahydrat und 401 mg (0,47 mmol) Triphosphopyridinnucleotid Natriumsalz (NADP⁺, 90%, z. B. FLUKA Nr. 93210) gegeben. Die erhöhte Konzentration an Magnesiumionen ist in diesem speziellen Anwendungsbeispiel beabsichtigt. Anschliessend wird die Reaktion durch die Zugabe von 1000 U (s. o.) rekLBADH gestartet, und der Reaktionskolben wird mit einem Stopfen verschlossen. Die Temperatur der Reaktionslösung wird während der Reaktion auf 25°C gehalten. Nach 16 Stunden wird der Kolbeninhalt filtriert und in einen Scheidetrichter überführt und mit 400 ml Essigsäureethylester extrahiert. Zur Erleichterung der Phasentrennung werden 135 g Natriumchlorid zugelöst. Die Phasen werden getrennt, und die wäßrige Phase wird noch zweimal mit jeweils 400 ml Essigsäureethylester extrahiert. Die vereinigten organischen Phasen werden mit Natriumsulfat getrocknet, filtriert und im Membranpumpenvakuum am Rotationsverdampfer bei maximal 40°C soweit wie möglich eingeengt. Man erhält 1,97 g öliges Rohprodukt (88%), das gemäß ¹H-NMR zu mindestens 90% aus dem gewünschten Produkt besteht. Als Verunreinigunge kann im Kernresonanzspektrum eine geringe Mengen eines Cyclisierungsproduktes (2-(t-Butyloxycarbonyl)-methyl-3(2H)-furanon) ausgemacht werden. Der Anteil an Cyclisierungsprodukt steigt, wenn die Reaktion bei höheren pH-Werten durchgeführt wird. Die Reinigung des gewünschten Produktes erfolgt durch Flash-Chromatographie (Säule mit 5 cm Durchmesser, 148 g Kieselgel, Essigsäureethylester : i-Hexan 4 : 6, 50 ml Fraktionen) und ergibt 1,67 g analytisch reines Produkt (75%) neben 0,16 g einer Mischfraktion mit dem oben erwähnten Cyclisierungsprodukt.

¹H-NMR (300 MHz, CDCl₃, 22°C, *es werden nur die Signale der Ketoform angegeben)* δ: 4.31 (m, 1H, CHOH), 3.62 (dd, J = 11.2, 5.1 Hz, 1H, H6), 3.57 (dd, J = 11.2, 5.0 Hz, 1H, H6), 3.41 (s, 2H, H2), 3.10 (s, 1H, OH, br), 2.90 (dd, J = 17.5, 5.0 Hz, 1H, H4), 2.83 (dd, J = 17.5, 7.3 Hz, 1H, H4), 1.47 (s, 9H, 3 x CH₃).- Keto : *Enol =* ca. 95 : 5.- ¹³C-NMR (75.5 MHz, CDCl₃, es werden *nur die Signale der Ketoform angegeben) δ:* 28.13 (3 x CH₃), 46.57 (C6), 48.43 (C4), 51.31 (C2), 67.58 (C5), 82.73 (OC(CH₃)₃), 166.22 (COO*t*Bu), 202.93 (C3).-
[α]²⁵_{D} = -24,9 (CHCl₃, c = 1,35)

Die Bestimmung der optischen Reinheit und der Konfiguration erfolgt durch Vergleich der spezifischen Drehung des Produktes mit der spezifischen Drehung einer möglichst enantiomerenreinen Probe derselben Verbindung. Dazu wird nach einem bekannten Verfahren (J. K. Thottathil, Y. Pendri, W. S. Li, D. R. Kronenthal, *US 5,278,313,* **1994),** ausgehend von einer kommerziell erhältlichen enantiomerenreinen Verbindung ((*S*)-4-Chlor-3-hydroxybutansäureethylester, 96%, ALDRICH Nr. 460524, > 97% ee (Herstellerangaben), gemessen: [α]²⁵_{D} = -21,7 (CHCl₃, c = 2,33), Lit.: [α]²¹_{D} = +20,9 (CHCl₃, c = 7,71), 97% ee, (R)-Enantiomer (M. Kitamura, T. Ohkuma, H. Takaya, R. Noyori, *Tetrahedron* Lett. **1988,** *29,* 1555-1556)), eine authentische Probe des Produktes (*S*)-6-Chlor-5-hydroxy-3-oxohexansäure-1,1-dimethylethylester hergestellt. Die Reinigung des so erhältlichen Rohproduktes erfolgt nach der oben angegebenen Methode.

Das ¹H- und ¹³C-Spektrum dieser Vergleichsverbindung stimmt exakt mit den oben angegebenen Daten überein. Die spezifische Drehung der gereinigten authentischen Probe beträgt:
[α]²⁵_{D} = -23,0 (CHCl₃, c = 1.52)

Ein Vergleich der beiden spezifischen Drehungen zeigt, daß die oben beschriebene enzymatische Reduktion ein Produkt höchster optischer Reinheit liefert. Die Übereinstimmung der Vorzeichen der spezifischen Drehung beweist, daß das Produkt in der (S)-Konfiguration vorliegt.

### c) (3R,5S)-6-Chlor-3,5-dihydroxyhexansäure-1,1-dimethylethylester

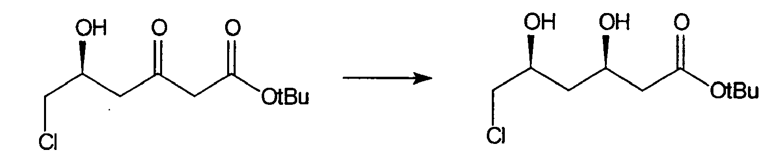

Ausgehend von dem in Schritt b) hergestelltem Rohprodukt kann die gewünschte Zielverbindung durch Reduktion mit Natriumborhydrid in Anwesenheit von Diethylmethoxyboran dargestellt werden. Dieses Verfahren ist speziell für das hier vorliegende Anwendungsbeispiel in der Literatur beschrieben (J. K. Thottathil, Y. Pendri, W. S. Li, D. R. Kronenthal, *US 5,278,313,* **1994).**

### Beispiel 2

### (3R,5R)-Dihydroxyhexansäure-1,1-dimethylethylester

### a) 3,5-Dioxohexansäure-1,1-dimethylethylester

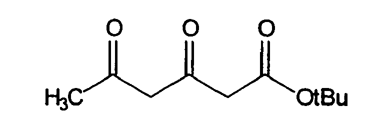

Diese prochirale Ausgangsverbindung wird nach einem in der Literatur beschriebenen Verfahren durch Alkoholyse und Decarboxylierung von acetoacetylierter Meldrumsäure dargestellt (F. Yuste, F. K. Brena, H. Barrlos, R. Sanchez-Obregon, B. Ortiz, F. Walls, *Synth. Commun.* **1988**, 18, 735-739).

### b) (R)-5-Hydroxy-3-oxohexansäure-1,1-dimethylethylester

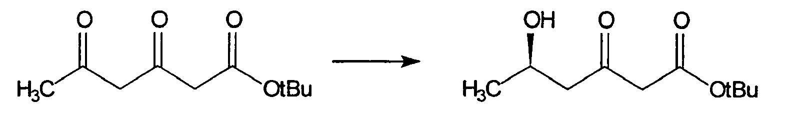

Für diese enzymatische Reaktion wird ein Zellrohextrakt verwendet, wie er bei Beispiel 1 Schritt b) beschrieben ist.

Zu 260 ml Phosphat-Puffer (125 mM Na₂HPO₄, 125 mM NaH₂PO₄, mit HCl auf pH 6,6 eingestellt) wird eine Lösung aus 1,04 g (5,17 mmol) des in Schritt a) hergestellten 3,5-Dioxohexansäure-1,1-dimethylethylester in 3,1 g (52 mmol) Isopropanol (zur Cofaktorregenerierung) gegeben. Weiterhin werden zu dieser Lösung 53 mg (0,26 mmol) Magnesiumchlorid-Hexahydrat und 220 mg (0,26 mmol) Triphosphopyridinnucleotid Natriumsalz (NADP⁺, 90%, FLUKA Nr. 93210) gegeben. Anschließend wird die Reaktion durch die Zugabe von 215 U rekLBADH gestartet, und der Reaktionskolben wird mit einem Stopfen verschlossen. Die Reaktionslösung wird langsam (ca. 60 rpm) mit einem Magnetrührer gerührt. Die Temperatur der Reaktionslösung wird während der Reaktion auf 25°C gehalten. Nach 28 Stunden wird der Kolbeninhalt durch eine Glasfritte (Porengröße 4) filtriert. Das Filtrat wird in einen Scheidetrichter überführt und mit 150 ml Essigsäureethylester extrahiert. Zur Erleichterung der Phasentrennung werden 75 g Natriumchlorid zugelöst. Die Phasen werden getrennt, und die wäßrige Phase wird noch zweimal mit jeweils 150 ml Essigsäureethylester extrahiert. Die vereinigten organischen Phasen werden mit Natriumsulfat getrocknet, filtriert und im Membranpumpenvakuum am Rotationsverdampfer bei maximal 40°C soweit wie möglich eingeengt. Man erhält 0,96 g öliges Rohprodukt (92%), das gemäß ¹H-NMR zu mindestens 90% aus dem gewünschten Produkt besteht. Als Verunreinigung kann im Kernresonanzspektrum eine geringe Menge des Eduktes ausgemacht werden. Die Reinigung des Produktes kann durch Vakuum-Kugelrohrdestillation (55°C, 0,02 mbar) erfolgen, oder aber durch Flash-Chromatographie. Mit der letzteren Methode können 0,82 g (78 %) des analytisch reinen Produktes gewonnen werden (Säule mit 3 cm Durchmesser, 45 g Kieselgel, Essigsäureethylester : n-Hexan 4 : 6, 20 ml Fraktionen). Die NMR-Daten stimmen mit den Literaturangaben überein (L. Shao, H. Kawano, M. Saburi, Y. Uchida, *Tetrahedron* **1993**, 49, 1997-2010).

¹H-NMR (300 MHz, CDCl₃, 22°C, es *werden nur die Signale der Ketoform angegeben)* δ: 4.27 (m, 1H, CHOH), 3.38 (s, 2H, H2), 2.91 (s, 1H, OH, br), 2.74 (dd, J = 17.7, 3.2 Hz, 1H, H4), 2.64 (dd, J = 17.7, 8.5 Hz, 1H, H4), 1.48 (s, 9H, 3 x CH₃), 1.21 (d, J = 6.4 Hz, 3H, H6).- *Keto : Enol =* ca. 95 : 5.- ¹³C-NMR (75.5 MHz, CDCl₃, es *werden nur die Signale der Ketoform angegeben)* δ: 22.59 (C6), 28.13 (3 x CH₃), 51.19, 51.29 (C2, C4), 63.93 (C5), 82.44 (OC(CH₃)₃), 166.42 (COO*t*Bu), 204.38 (C3).-
[α]²⁶_{D} = -40.5 (c = 1.15, CHCl₃)

Durch Vergleich der spezifischen Drehung des Produktes aus der enzymatischen Reduktion mit dem Literaturwert für das enantiomerenreine Produkt ist ersichtlich, daß die vorliegende Reaktion ein (*R*)-konfiguriertes Produkt höchster optischer Reinheit liefert (Lit.: [α]²⁶_{D} = -39.6 (c = 2, CHCl₃), 99% ee (P. F. Deschenaux, T. Kallimopoulos, H. S. Evans, A. Jacot-Guillarmod, *Helv. Chim. Acta* 1989, 72, 731-737).

### c) (3R,5R)-Dihydroxyhexansäure-1,1-dimethylethylester

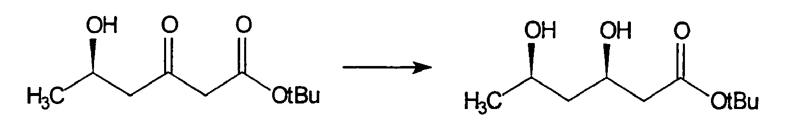

Ausgehend von dem in Schritt b) hergestellten *(R)*-5-Hydroxy-3-oxohexansäure-1,1-dimethylethylester kann die gewünschte Zielverbindung durch Reduktion mit Natriumborhydrid in Anwesenheit von Diethylmethoxyboran dargestellt werden. Dieses Verfahren ist speziell für das hier vorliegende Anwendungsbeispiel in der Literatur beschrieben (C. Masoni, P. F. Deschenaux, T. Kallimopoulos, A. Jacot-Guillarmod, *Helv. Chim. Acta* **1989,** *72,* 1284-128).

## Patentansprüche

1. Verfahren zur r-selektiven und regioselektiven Reduktion in Position 5 von 3,5 Dioxocarbonsäuren, deren Salze und Ester der allgemeinen Formel 4, 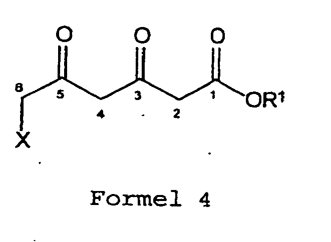 bei deren R¹ eine Komponente aus der Gruppe Alkyl, Alkenyl, Cycloalkyl, Cycloalkenyl, Aryl, Aralkyl, Cycloalkylalkyl, Wasserstoff oder Metallkation ist und X eine Komponente aus der Gruppe Wasserstoff, Halogen, Alkyl, Aryl, CH=CHR², C≡CR³ (mit R² = R¹ außer Metallkation und R³ = R¹) ist,
**dadurch gekennzeichnet,**
**dass** die Reduktion mittels einer Alkoholdehydrogenase in Anwesenheit eines Cofaktors katalysiert wird.

2. Verfahren nach Anspruch 1,
**dadurch gekennzeichnet,**
**daß** die Alkoholdehydrogenase rekombinant ist.

3. Verfahren nach Anspruch 1 oder 2,
**dadurch gekennzeichnet,**
**daß** die Alkoholdehydrogenase aus *Lactobacillus* stammt.

4. Verfahren nach Anspruch 3,
**dadurch gekennzeichnet,**
**daß** die Alkoholdehydrogenase aus Lactobacillus brevis stammt

5. Verfahren nach einem der Ansrüche 1 bis 4,
**dadurch gekennzeichnet,**
**daß** die Alkoholdehydrogenase in *Escherichia coli* überexprimiert ist.

6. Verfahren nach einem der Ansprüche 1 bis 5,
**dadurch gekennzeichnet,**
**daß** das Halogen Fluor oder Chlor ist.

7. Verfahren nach einem der Ansprüche 1 bis 6,
**dadurch gekennzeichnet,**
**daß** der Rest R¹ eine Komponente aus der Gruppe von Methyl, Ethyl, n-Propyl, i-Propyl, Allyl, t-Butyl Pentyl, i-Pentyl, n-Hexyl, i-Hexyl, Vinyl, 1-Propenyl, Butenyl, i-Butenyl, Cyclopropyl, Cyclobutyl, Cyclopentyl, Cyclohexyl, Cycloheptyl, Cyclopentenyl, Cyclohexenyl, Cycloheptenyl, Cyclooctenyl, Phenyl, p-Tolyl, Furanyl und Benzyl ist.

8. Verfahren nach einem der Ansprüche 1 bis 7,
**dadurch gekenzeichnet,**
daß es bei Raumtemperatur durchgeführt wird.

9. Verfahren nach einem der Ansprüche 1 bis 7
**dadurch gekennzeichnet,**
**daß** es bei einem pH-Wert von 5,5 durchgeführt wird.

10. Verfahren nach einem der Ansprüche 1 bis 9,
**dadurch gekennzeichnet,**
**daß** das Reaktionsprodukt in der 3-Position hochdiastereoselektiv zu einem syn-, oder anti-Diol reduziert wird.

11. Verfahren nach Anspruch 10,
**dadurch gekennzeichnet,**
**daß** die diastereoselektive Reduktion mittels eines Enzyms erfolgt.

12. Verfahren nach einem der Ansprüche 1 bis 11,
**dadurch gekennzeichnet,**
**daß** es in einem Enzym-Membranreaktor durchgeführt wird.

13. Verfahren nach einem der Ansprüche 1 bis 12,
**dadurch gekennzeichnet,**
**daß** die prochiralen Ausgangsverbindungen nach Formel 4 ausgehend von Dilithio-Bisenolaten nach Formel A 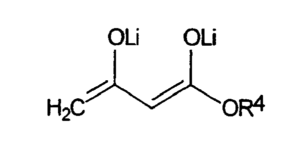 mit R⁴ = Alkyl, Alkenyl, Cycloalkyl, Cycloalkenyl, Aryl, Aralkyl, Cycloalkylalkyl oder Metallkation durch Acylierung mit Carbonsäureestern nach Formel B 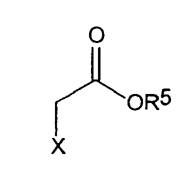 mit R⁵ = Alkyl dargestellt werden.

14. Verfahren nach Anspruch 13,
**dadurch gekennzeichnet,**
**daß** es bei einer Temperatur zwischen -72 und -65 °C durchgeführt wird.

15. Verfahren nach Anspruch 13 oder 14,
**dadurch gekennzeichnet,**
**daß** es in einem aprotischen und koordinierenden Lösungsmittel durchgeführt wird.

## Claims

1. Method for r-selective and regio-selective reduction in Position 5 of 3.5 dioxocarboxylic acids, its salts and esters of the general Formula 4, 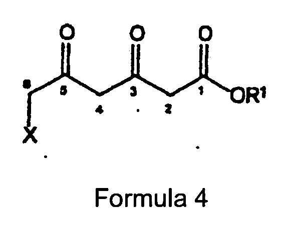 the R¹ of which is a component of the group alkyl, alkenyl, cycloalkyl, cycloalkenyl, aryl, aralkyl, cycloalkylalkyl, hydrogen or metalcation, and X is a component of the group nitrogen, halogen, alkyl, aryl, CH=CHR², C≡CR³ (with R² = R¹ apart from metalcation and R³ = R¹)
**characterised in that**
the reduction is catalysed by means of an alcohol dehydrogenase in the presence of a co-factor.

2. Method according to Claim 1,
**characterised in that**
the alcohol dehydrogenase is re-combinant.

3. Method according to Claim 1 or 2,
**characterised in that**
the alcohol dehydrogenase originates from *Lacto Bacillus.*

4. Method according to Claim 3,
**characterised in that** the alcohol dehydrogenase originates from *Lacto Bacillus.*

5. Method according to one of Claims 1 to 4,
**characterised in that**
the alcohol dehydrogenase in *Escherichia Coli* is overexprimed.

6. Method according to one of Claims 1 to 5,
**characterised in that**
the halogen is fluor or chlorine.

7. Method according to one of Claims 1 to 6,
**characterised in that**
the remainder R¹ is a component of the group of methyl, ethyl, n-propyl, i-propyl, allyl, t-butyl, pentyl, i-pentyl, n-hexyl, i-hexyl, vinyl, 1-propenyl, butenyl, i-butenyl, cyclopropyl, cyclobutyl, cyclopentyl, cyclohexyl, cycloheptyl, cyclopentenyl, cyclohexenyl, cycloheptenyl, cyclooctenul, phenyl, p-tolyl, furanyl and benzyl.

8. Method according to one of Claims 1 to 7,
**characterised in that**
it is carried out at room temperature.

9. Method according to one of Claims 1 to 7,
**characterised in that**
it is carried out at a pH-value of 5.5.

10. Method according to one of Claims 1 to 9,
**characterised in that**
the reaction product in the 3-position is high-diastereoselectively reduced to a syn- or anti-diol.

11. Method according to Claim 10,
**characterised in that**
the diastereoselectve reduction is carried out by means of an enzyme.

12. Method according to one of Claims 1 to 11,
**characterised in that**
it is carried out in an enzyme membrane reactor.

13. Method according to one of Claims 1 to 12,
**characterised in that**
the prochiral base connections are shown according to Formula 4 based on dilithio-bisenolates according to Formula A 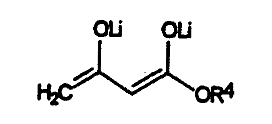 with R⁴ = alkyl, alkenyl, cycloalkyl, cycloalkenyl, aryl, aralkyl, cycloalkylalkyl or metalcation by acylisation with carboxylic acid esters according to Formula B 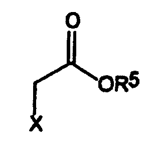 with R⁵ = alkyl.

14. Method according to Claim 13,
**characterised in that**
it is carried out at a temperature between -72 and -65°.

15. Method according to Claim 13 or 14,
**characterised in that**
it is carried out in an aprotic and co-ordinating solution medium.

## Revendications

1. Procédé de réduction r-sélective et régio-sélective en position 5 d'acides 3,5 dioxocaboxyliques, de leurs sels et de leurs esters de formule développée 4, 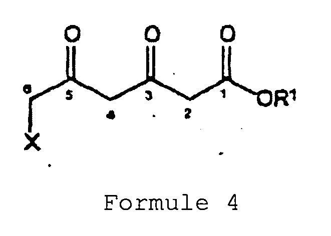 dans lesquels R¹ est un constituant choisi dans le groupe alcoyle, alcényle, cycloalcoyle, cycloalcényle, aryle,aralcoyle, cycloalcoylalcoyle, hydrogène ou cation métallique et X est un constituant du groupe hydrogène, halogène, alcoyle, aryle, CH=CHR², C≡CR³ (avec R² = R¹ à l'exception de cation métallique et R³ = R¹),
**caractérisé en ce que** l'on catalyse la réduction au moyen d'une alcooldéshydrogénase en la présence d'un cofacteur.

2. Procédé suivant la revendication 1,
**caractérisé**
**en ce que** l'alcool déshydrogénase est recombinante.

3. Procédé suivant la revendication 1 ou 2,
**caractérisé**
**en ce que** l'alcooldéshydrogénase provient de *Lactobacillus.*

4. Procédé suivant la revendication 3,
**caractérisé**
**en ce que** l'alcooldéshydrogénase provient de *Lactobacillus brevis.*

5. Procédé suivant l'une des revendications 1 à 4,
**caractérisé**
**en ce que** l'alcooldéshydrogénase est surexprimée dans *Escherichia coli.*

6. Procédé suivant l'une des revendications 1 à 5,
**caractérisé**
**en ce que** l'halogène est le fluor ou le chlore.

7. Procédé suivant l'une des revendications 1 à 6,
**caractérisé**
**en ce que** le radical R¹ et un constituant choisi dans le groupe constitué de méthyle, éthyle, n-propyle, i-propyle, allyle, t-butyl pentyle, i-pentyle, n-hexyle, i-hexyle, vinyle, 1-propényle, butényle, i-butényle, cyclopropyle, cyclobutyle, cyclopentyle, cyclohexyle, cycloheptyle, cyclopentényle, cyclohexényle, cycloheptényle, cyclooctényle, phényle, p-tolyle, furanyle et benzyle.

8. Procédé suivant l'une des revendications 1 à 7,
**caractérisé**
**en ce que** on l'effectue la température ambiante.

9. Procédé suivant l'une des revendications 1 à 7,
**caractérisé**
**en ce que** on l'effectue à un pH de 5,5.

10. Procédé suivant l'une des revendications 1 à 9,
**caractérisé**
**en ce que** l'on réduit le produit de réaction en la position 3 d'une manière très diastéréosélective en un diol syn ou en un diol anti.

11. Procédé suivant la revendication 10,
**caractérisé**
**en ce que** on l'effectue la réduction diastéréosélective au moyen d'un enzyme.

12. Procédé suivant l'une des revendications 1 à 11,
**caractérisé**
**en ce que** on l'effectue dans un réacteur à membrane à enzyme.

13. Procédé suivant l'une des revendications 1 à 12,
**caractérisé**
**en ce que** l'on prépare les composés de départ prochiraux suivant la formule 4 à partir de dilithio-bisénolates de formule A 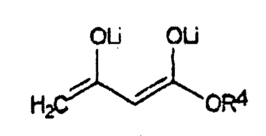 avec R⁴ = alcoyle, alcényle, cycloalcoyle, cycloalcényle, aryle, aralcoyle, cycloalcoylalcoyle ou cation métallique par acylation par des esters carboxyliques de formule B 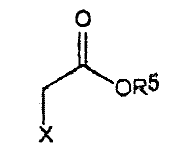 avec R⁵ = alcoyle.

14. Procédé suivant la revendication 13,
**caractérisé**
**en ce que** on l'effectue à une température comprise entre -72 et -65°C.

15. Procédé suivant la revendication 13 ou 14,
**caractérisé**
**en ce que** on l'effectue dans un solvant aprotique et de coordination.
